# EUROPEAN PATENT APPLICATION

(11) **EP 2 314 162 A1**
(43) Date of publication of application: **27.04.2011**
(21) Application number: 10179983.1
(22) Date of filing: 11.01.2006
(51) Int. Cl.: A01N 43/40, A01P 1/00, A61K 8/49, A61K 8/33, A61K 8/34, A61Q 19/10, A01N 31/02

(54) **Alcoholic compositions for disinfection**

(30) Priority: 19.01.2005 DE 102005002645
(62) Divisional of application: 06100226.7
(71) Applicant: Air Liquide Santé (International), 75007 Paris (FR); Schuelke & Mayr, 22851 Norderstedt (DE)
(72) Inventor: Beilfuss, Wolfgang, 22339 Hamburg (DE); Behrends, Sabine, 25482 Appen (DE); Goroncy-Bermes, Peter, 22145 Hamburg (DE); Puchstein, Burghard, 20257 Hamburg (DE)
(74) Representative: Beroud, Amandine

(57) **Abstract**

The invention relates to alcoholic compositions which include a) 1-(2-ethylhexyl)glycerol ether, b) octenidine dihydrochloride, and c) one or more aliphatic alcohols, and to the use of the compositions for the disinfection of animate and inanimate surfaces, in particular for surgical and hygienic hand disinfection and disinfectant handwashing.

## Description

The present invention relates to alcholic compositions and their use for disinfecting animate and inanimate surfaces, in particular for surgical and hygienic hand disinfection and disinfectant handwashing.

The aim of hand disinfection, hand decontamination and skin antisepsis is to prevent transmission of microorganisms and viruses or to suppress their unwanted introduction into endangered regions of the body or more sensitive regions. Compositions for hygienic hand disinfection and for disinfectant handwashing must satisfy certain efficacy requirements, some of which are defined in standards. Various methods are possible for treating the hands after contamination.

Hygienic hand disinfection complying with EN 1500 as rubbing method without addition of water causes the death or inactivation of the transient microorganisms on the hands, without any risk of microbes being disseminated in the surroundings and without any risk of recontamination of the hands by microorganisms possibly present in the water. Disinfectant handwashing complying with EN 1499 with a microbicidal composition using tap water is likewise directed against transient microorganisms without precluding their dissemination in the surroundings. It serves in particular to reduce microbes during the washing procedure, but cannot replace hand disinfection. In disinfectant handwashing, the composition is rubbed in undiluted and foamed with a little water, and the hands are cleaned and thoroughly rinsed with water.

Compositions for disinfectant handwashing and hygienic hand disinfection must be effective after acting for short times (e.g. 30 seconds or 1 minute). In the efficacy tests, it is important that there is a good effect (RF > 3 orders of magnitude) after acting for these short times. For toxicological reasons it is additionally necessary for, besides adequate efficacy, in particular the compatibility with human skin to be ensured, even if the intended use is on inanimate surfaces.

Commercially available compositions for disinfectant handwashing are usually alcohol- or surfactant-containing liquid soaps and wash lotions ready for use with further biocidal agents added. Known compositions comprise as biocidal agents for example short-chain alcohols and as excipients superfatting agents, moisturizers and fragrances to improve the skin compatibility and acceptance. To enhance the efficacy and to achieve a residual effect intended to prevent the number of microbes on the hands increasing again, the known compositions often also comprise additional agents such as biguanides (e.g. chlorhexidine), quaternary ammonium compounds (e.g. benzalkonium chloride), phenol derivatives (e.g. ortho-phenylphenol) or carboxylic acids. For example, a known alcholic hand disinfection composition includes about 53% by weight propanols and about 0.8% by weight chlorhexidine digluconate, and excipients. A further known alcholic hand disinfection composition includes approximately 75% by weight ethanol, 0.1% by weight 2-biphenylol, polyvidone 30 and excipients.

The known compositions for disinfectant handwashing have a number of disadvantages. Thus, some products show an efficacy which is not always satisfactory, or the desired efficacy is achieved only after acting for a lengthy time. Some compositions additionally have insufficient skin compatibility. Thus, compositions based on chlorhexidine are reported to be prone to skin incompatibility, it being suggested for example that there is partial release of chloroaniline.

An additional factor is that agents with organically bound halogen such as chlorhexidine have only conditional environmental compatibility. Chlorhexidine is moreover sufficiently effective only with a comparatively high concentration of the agent (e.g. 2% by weight) in wash products and may lead to discolorations on contaminated surfaces. Poly(hexamethylenebiguanide) hydrochloride is a polymeric biguanide salt whose structure is not exactly defined. For this reason, no medicinal product with this agent yet has marketing authorization in Germany. The polymeric biguanide may be employed only in a concentration of up to 0.3% by weight in cosmetic compositions.

Hence there is a need for compositions with improved microbicidal efficacy and, at the same time, good skin compatibility for used in disinfection.

It has now surprisingly been found that the problems of the prior art are solved by alcoholic compositions which include a) 1-(2-ethylhexyl) glycerol ether, b) octenidine dihydrochloride, and c) one or more aliphatic alcohols.

Compositions preferred in this connection are those free of aromatic alcohols such as 2-phenoxyethanol, benzyl alcohol, phenethyl alcohol, 1-phenoxypropan-2-ol, 3-(4-chlorophenoxy)-1,2-propanediol, chlorobutanol, 2,4-dichlorobenzyl alcohol or mixtures thereof. Preferred compositions are moreover those including no salts selected from the salts of benzoic acid, propionic acid, salicylic acid, sorbic acid, 4-hydroxybenzoic acid, dehydroacetic acid and 10-undecylenic acid and/or any of the free acids mentioned.

### a) 1-(2-ethylhexyl)glycerol ether

The content of 1-(2-ethylhexyl)glycerol ether is generally in the range from 0.02 to 2% by weight, preferably 0.04 to 1% by weight, more preferably 0.08 to 1% by weight, such as 0.15 to 0.5% by weight, for example about 0.25% by weight.

### b) Octenidine dihydrochloride

In a preferred embodiment, the content of octenidine dihydrochloride is in the range from 0.01 to 2% by weight, more preferably 0.01 to 1% by weight, in particular 0.02 to 0.5% by weight, such as 0.05 to 0.2% by weight, for example about 0.1% by weight.

### c) Aliphatic alcohols

In principle, all suitable aliphatic alcohols can be employed according to the invention, preferably linear and branched C₁ to C₁₀ alcohols. For example ethanol, isopropanol and n-propanol as well as mixtures thereof are preferably employed, ethanol being particularly preferred in all embodiments of the invention.

In a first preferred embodiment the content of aliphatic alcohol in the compositions according to the invention is in the range from 2 to 99% by weight, more preferably 15 to 98.5% by weight, in particular 30 to 98% by weight, such as 50 to 95% by weight.

In a second preferred embodiment, the content of aliphatic alcohol in the compositions according to the invention is more than 40% by weight, more preferably more than 41% by weight, in particular more than 42% by weight, for example more than 45% by weight, such as more than 50% by weight, more preferably more than 55% by weight, particularly preferably 60 to 90% by weight or 70 to 85% by weight.

In a further preferred embodiment the compositions according to the invention comprise one or more quarternary ammonium compounds. It is possible in principle to employ according to the invention all suitable quaternary ammonium compounds. The quaternary ammonium compound is preferably selected from betaines and dimethylammonium salts.

Quaternary ammonium salts employed according to the invention are represented by the formula [R¹R²R³(CH₃)N]⁺[X]⁻, where R¹ to R³ may be identical or different and are selected from C₁- to C₃₀-alkyl, aralkyl, -alkenyl and mixed groups which may have one or more atoms selected from O, S, N and P, where R¹ to R³ are for example C₈- to C₁₈-alkyl, benzyl or methyl, preferably C₉- to C₁₈-alkyl, benzyl or methyl, such as C₁₆-alkyl, benzyl or methyl. X is an anion (of an inorganic or organic acid). It is possible in this connection for both anion and cation of the quaternary ammonium salt to be multiply charged ions, resulting in a stoichiometry [A⁽ⁿ⁺⁾]ₘ[K^{(m+)}]ₙ.

Suitable quaternary ammonium salts according to the invention are all quaternary ammonium salts of the abovementioned formula which are known in the art, as disclosed for example in WO 00/63337, which is incorporated herein by reference. However, dialkyldimethylammonium salts are preferably employed, for example dialkyldimethylammonium chlorides whose alkyl chains are selected independently of one another from C₈- to C₁₈-alkyl, preferably C₉- to C₁₈-alkyl, such as C₁₆-alkyl. One of the methyl groups in the dialkyldimethylammonium salts may be an alkoxylated, for example ethoxylated, hydroxymethyl group.

Quaternary ammonium salts which are preferably employed according to the invention are compounds of the formulae [R¹N(CH₃)₃]⁺[X]⁻, [R¹R²N(CH₃)₂]⁺[X]⁻ and [R¹R²R³ (CH₃) N] ⁺ [X] ⁻, where R¹ to R³ are selected independently of one another from C₈- to C₁₈-alkyl and -(CH₂-CHR⁴O)ₙ-R⁵, where n is a number from 1 to 20, preferably 1 to 5, and R⁴ and R⁵, which may be identical or different, are H and/or C₁- to C₄-alkyl, preferably H.

Examples of anions and classes of anions of the quaternary ammonium salts employed according to the invention are hydroxide, sulphate, hydrogensulphate, methosulphate, ethosulphate, lauryl sulphate, lauryl ether sulphate, cellulose sulphate, sulphamate, halide (fluoride, chloride, bromide, iodide), nitrite, nitrate, carbonate, hydrogencarbonate, phosphate, alkyl phosphate, metaphosphate, polyphosphate, thiocyanate, carboxylic salt such as benzoate, lactate, acetate, propionate, citrate, succinate, glutarate, adipate, toluenesulphonate (tosylate) and salicylate. Particularly preferred anions are chloride and propionate.

The quaternary ammonium salts which are particularly preferably employed are mecetronium etilsulfate (hexadecyl(ethyl)dimethylammonium ethyl sulphate) and benzalkonium chloride.

All suitable betaines can be employed as betaine. Cocamidopropylbetaine is particularly preferred.

A preferred amount of quaternary ammonium compound is in the range from 0.01 to 5% by weight. The amount of betaine is preferably in the range from 0.03 to 5% by weight, more preferably 0.05 to 3% by weight, in particular 0.1 to 1% by weight, such as 0.15 to 0.5% by weight, and is for example about 0.3% by weight. The amount of quaternary ammonium salt (for example mecetronium etilsulfate or benzalkonium chloride) is preferably from 0.01 to 5% by weight, more preferably 0.02 to 2.5% by weight, in particular 0.04 to 0.8% by weight, such as 0.05 to 0.4% by weight.

Moreover, compositions which are preferred also include 1 to 5% by weight, preferably 2 to 3% by weight of superfatting agents, such as polyol fatty acid esters, e.g. glycerol esters, preferably glycerol cocoate, isopropyl myristate, isopropyl palmitate and triglycerides.

Compositions which are additionally preferred are those additionally including from 0.05 to 0.8% by weight, more preferably 0.1 to 0.5% by weight of skin care additives such as allantoin, glycerol or sodium gluconate.

The compositions of the invention are preferably present in aqueous form and comprise from 1 to 80% by weight, more preferably 2 to 65% by weight, in particular 5 to 50% by weight, such as 8 to 40% by weight, 10 to 35% by weight, 12 to 30% by weight or 15 to 22% by weight of water, based on the composition. In a preferred embodiment the water content of the compositions according to the invention is less than 55% by weight, more preferably less than 50% by weight, in particular less than 40% by weight.

A particularly preferred composition includes:
a) 0.1 to 0.5% by weight, such as about 0.25% by weight 1-(2-ethylhexyl)glycerol ether,
b) 0.05 to 0.2% by weight, such as about 0.1% by weight octenidine dihydrochloride and
c) 60 to 90% by weight, such as about 79 or about 83% by weight of one or more aliphatic alcohols, for example ethanol, and where appropriate
d) 0.1 to 0.5% by weight, such as about 0.25% by weight glycerol,
e) 0.5 to 2% by weight, such as about 1.0% by weight isopropylmyristate,
f) 0.4 to 2% by weight, such as about 0.8% by weight myristyl alcohol and/or
g) 0.1 to 0.5% by weight, such as about 0.3% by weight cetearyl octanoate, and
h) water as the remainder.

The composition may additionally comprise functional additives such as colorants, perfume, buffers, electrolytes and moisturizing factors. The preferred pH of the composition is from 4 to 8.

The invention further relates to the use of the abovementioned compositions for disinfection or decontamination of animate (e.g. skin, hands, mucous membrane, wounds) and inanimate surfaces (e.g. apparatuses, instruments, endoscopes). The compositions employed as products for personal hygiene and as medical wash products, high-value, soap-free wash products for all hand, skin and body washing and as bath additive.

The compositions according to the invention are used in particular for hygienic hand disinfection or for disinfectant handwashing, in particular as skin antiseptic. The compositions according to the invention are suitable for hygienic and surgical hand disinfection, hygienic handwashing, hand decontamination, skin decontamination, personal hygiene washing lotion, as antimicrobial washing lotion, for (whole) body washing and care in connection with MRS (methicillin-resistant Staphylococcus aureus) and furthermore for disinfectant handwashing, for hygienic catheter care in patients, as handwashing product such as, for example, as antimicrobial soap, handwashing gel or handwashing lotion. The compositions can be used advantageously in all sectors with enhanced hygiene requirements in the medical and nonmedical sector, e.g. hospitals, medical practices, old people's and nursing homes, and in the food products and kitchen sectors. The invention relates in a particularly preferred embodiment to the use of the compositions for surgical and hygenic hand disinfection and disinfectant handwashing.

The compositions can additionally also be formulated as gel compositions, ointment compositions and antimicrobial coatings and have an excellent effect with, at the same time, good skin compatibility and stability. The compositions show a distinctly improved efficacy compared with known compositions. Synergistic increases in effect are observed here in some cases. The possible inhibition of the efficacy of octenidine dihydrochloride, which is observed with many surfactants, does not occur with aliphatic alcohols. The excellent skin compatibility of the compositions is also to be emphasized.

It was surprising that a disinfectant effect is achieved after only a short time with alcoholic compositions according to the invention containing 1-(2- ethylhexyl) glycerol ether. Although glycerol monoalkyl ethers are known, for example from DE 42 40 674 C1, to act as deodorant agents, disinfection is immaterial for deodorants, since known deodorant agents should merely have antimicrobial activity, whereas hand disinfectants must have microbicidal activity. An additional factor is that 1-(2- ethylhexyl) glycerol ether on their own has virtually no microbicidal effect, and accordingly is unsuitable as (sole) agents for hand disinfection. It was therefore surprising that 1- (2- ethylhexyl) glycerol ether contribute in the compositions according to the invention to the disinfectant effect.

In addition, modern deodorant compositions are preferably free of relatively large amounts of aliphatic alcohols. Thus, skin compatibility of deodorant agents in combination with relatively large amounts of aliphatic alcohols is immaterial in deodorant compositions. By contrast, it was surprising that compositions according to the invention can be formulated with large amounts of aliphatic alcohol in some cases, without the efficacy of the octenidine dihydrochloride and 1- (2- ethylhexyl) glycerol ether being impaired, and without skin incompatibility being observed.

The compositions according to the invention additionally have the following advantages:
- Good tolerability in combination with good antimicrobial efficacy and excellent short-term effect (acting for a time of, for example, 30 or 60 seconds), and very good effect on Gram-negative bacteria.
- Good efficacy for viruses (enveloped and non-enveloped) and multidrug-resistant microorganisms such as MRSA.
- No need to add a further preservative because they are self-preservative.
- The solubility in water and water-based compositions of 1-(2-ethylhexyl)glycerol ether is only limited (solubility in water 0.1% by weight). The presence of octenidine dihydrochloride in the compositions according to the invention leads to a significantly improved solubility in water of the 1-(2-ethylhexyl)glycerol ether.

The advantages of the invention are evident in particular from the following examples.

### Examples

The following compositions were formulated. All data are in % by weight.

| | A | B |
|---|---|---|
| Ethanol, methyl ethyl ketone - denatured | 79.00 | 83.00 |
| Octenidine dihydrochloride | 0.10 | 0.10 |
| Sensiva^{®} SC 50 | 0.25 | 0.25 |
| Isopropyl myristate | 1.00 | |
| Myristyl alcohol | | 0.80 |
| Cetearyl octanoate | 0.30 | |
| Medium-chain triglycerides | | 0.20 |
| Polyvidone K=30 | 0.20 | |
| Sorbitol solution | 0.50 | |
| Glycerol | | 0.25 |
| Water | ad 100 | ad 100 |

The good efficacy of the compositions according to the invention can be verified in the quantitative suspension test. Compare standard methods of the DGHM for testing chemical disinfection methods, J. Gebel, H.-P. Werner, A. Kirsch-Altena, K. Bansemir, mhp Verlag GmbH, Wiesbaden, Germany, method 9.1 (date: 1 September 2001) (quantitative suspension test with bacteria (apart from mycobacteria and fungi)).

## Claims

1. Alcoholic composition which includes
a) 1-(2-ethylhexyl)glycerol ether,
b) octenidine dihydrochloride, and
c) one or more aliphatic alcohols
the total amount of component c) being more than 40% by weight.

2. Composition according to claim 1, **characterized in that** it includes from 0.02 to 2% by weight 1-(2-ethylhexyl)glycerol ether.

3. Composition according to claim 1 or 2, **characterized in that** it includes from 0.01 to 2% by weight octenidine dihydrochloride.

4. Composition according to any of the preceding claims, **characterized in that** the aliphatic alcohol is ethanol, isopropanol or n-propanol, preferably ethanol.

5. Composition according to any of the preceding claims, **characterized in that** the composition includes from 50 to 99% by weight aliphatic alcohol.

6. Composition according to one of Claims 1 to 4, **characterized in that** the composition also includes from 1 to 50% by weight water.

7. Composition according to any of the preceding claims, **characterized in that** it includes
a) 0.1 to 0.5% by weight, such as about 0.25% by weight 1-(2-ethylhexyl)glycerol ether,
b) 0.05 to 0.2% by weight, such as about 0.1% by weight octenidine dihydrochloride and
c) 60 to 30% by weight, such as about 79 or about 83% by weight of one or more aliphatic alcohols, for example ethanol, and where appropriate
d) 0.1 to 0.5% by weight, such as about 0.25% by weight glycerol,
e) 0.5 to 2% by weight, such as about 1.0% by weight isopropylmyristate,
f) 0.4 to 2% by weight, such as about 0.8% by weight myristyl alcohol and/or
g) 0.1 to 0.5% by weight, such as about 0.3% by weight cetearyl octanoate, and
h) water as the remainder.

8. Use of an alcoholic composition which includes
a) 1-(2-ethylhexyl)glycerol ether,
b) octenidine dihydrochloride, and
c) one or more alihatic alcohols
for the disinfection of animate and inanimate surfaces.

9. Use of an alcoholic composition which includes
a) 1-(2-ethylhexyl)glycerol ether,
b) octenidine dihydrochloride, and
c) one or more aliphatic alcohols
for hygienic hand disinfection or for disinfectant handwashing.
